# EUROPEAN PATENT APPLICATION

(11) **EP 4 439 572 A1**
(43) Date of publication of application: **02.10.2024**
(21) Application number: 23164248.9
(22) Date of filing: 27.03.2023
(51) Int. Cl.: G16H 10/60, G16H 15/00

(54) **TRANSFERRING DATA BETWEEN CLINICAL INFORMATION SYSTEMS**

(71) Applicant: Koninklijke Philips N.V., 5656 AG Eindhoven (NL)
(72) Inventor: DE BLIEK, Hubrecht Lambertus Tjalling, 5656AG Eindhoven (NL); TRUYEN, Roel, 5656AG Eindhoven (NL)
(74) Representative: Philips Intellectual Property & Standards

(57) **Abstract**

A mechanism for transferring data from a first data record, in a source clinical information system, to a second data record, in a (different) destination clinical information system. A source data element, from the first data record, and a destination data element, from the second data record, are identified. A check is performed that both the first and second data records relate to a same clinical subject. Only if the check is passed is data transferred from the source data element to the destination data element.

## Description

### FIELD OF THE INVENTION

The present invention relates to the field of clinical information systems.

### BACKGROUND OF THE INVENTION

An increasingly important aspect in healthcare settings is the accurate documentation (in one or more clinical information systems) by clinicians, i.e., healthcare professionals, of information about a subject or patient. Inaccurate documentation of information has been known to lead to misdiagnosis and/or mistreatment, as other professionals rely upon accurate information in order to perform diagnostic or treatment tasks.

Currently, clinicians are required to enter data manually into data records in/of clinical information systems in order to document such information about a subject, e.g., findings, procedures, materials used, impressions and so on. This holds even if the relevant information can be found in another clinical information system, with the clinician needing to manually replicate the desired data.

In the context of the present disclosure, a clinical information system is a database system that stores one or more data records holding clinical information. Typically, different clinical information systems can be accessed via a same user interface, e.g., a same computer. It is common for each clinical information system to be associated with its own graphical user interface, to allow a clinician to directly interact with records of a specific clinical information system.

As previously pointed out, it is currently not always possible for data stored in one clinical information system to be exported to another clinical information system. As a result, clinicians have to enter the same information multiple times in different clinical information systems. This can lead to errors and inconsistencies in the data as a result of human error. This also creates a lot of waste and inefficiencies, and leads to clinician overload. Indeed, reporting demands have been identified as being a chief cause of clinician overload.

### SUMMARY OF THE INVENTION

The invention is defined by the claims.

According to examples in accordance with an aspect of the invention, there is provided a computer-implemented method for transferring data from a source clinical information system to a destination clinical information system.

The computer-implemented method comprises: identifying a desired source data element in a first data record in the source clinical information system; identifying a desired destination data element in a second data record in the destination clinical information system, wherein the desired source data element contains data for transferal to the desired destination data element; processing information in the first data record and information in the second data record to predict whether or not the first and second data records relate to a same clinical subject; responsive to predicting that the first and second data records relate to the same clinical subject, copying data in the desired source data element to the desired destination data element; and responsive to predicting that the first and second data records do not relate to the same clinical subject, preventing the copying of data in the desired source data element to the desired destination data element.

*The proposed approach therefore provides a mechanism for transferring data found in a data element of a first data record to a data element of a second data record. The data records are stored in separate or different clinical information systems. To reduce a risk of error, a check is performed that the first and second data records pertain to the same subject*/*individual, i.e., are data records of a same subject*/*individual. A reduced risk of error directly results in reduced risk of misdiagnosis, or incorrect analysis and*/*or treatment. This is because there is a reduced risk that information of one subject will be incorrectly attributed to another subject.*

*The proposed approach thereby provides a layer or liaison between two clinical information systems. In particular, herein suggested techniques facilitate automated or semi-automated transferal of data between data records of different clinical information systems.*

*In the context of the present disclosure, a clinical subject is an individual (human or animal) that is of clinical interest, e.g., a patient.*

The step of identifying the desired source data element comprises receiving a first user input indicating the desired source data element. *This approach facilitates a user -guided transferal mechanism, and allows an individual or user to select the data to be transferred. This improves a flexibility in transferring data from the source clinical information system to the destination clinical information system.*

In some examples, the first user input indicates a first region, of a graphical representation of the first data record, that graphically represents the desired source data element; and the step of copying data in the desired source data element comprises: processing the indicated first region to produce a first graphically-derived version of the data in the desired source data element; and copying the first graphically-derived version of the data into the desired destination data element.

*This approach facilitates a simple and intuitive user interaction with a graphical representation of the first data record. Thus, a user is not required to have an in-depth knowledge or require access to the raw data record in order to facilitate the transfer of data. Rather, a graphical representation of the data record can be used as a proxy for the raw data record.*

The step of processing the indicated region may comprise processing the indicated region using an optical character recognition technique. *This provides a technique for automated extraction of information contained in the first data record without requiring direct access to the first data record. This can facilitate, for instance, extraction of data from a data record for which a user has read-only access. This approach also avoids the copying of extraneous data of the desired source data element to the desired destination data element (e.g., metadata or the like), as the relevant clinical information to be replicated will be the information displayed or provided in graphical form. This can further reduce or avoid a risk of error down the line, e.g., reduce a risk that metadata is confused for potentially clinically relevant information.*

The computer-implemented method may further comprise a step of processing a graphical representation of the first data record to produce a plurality of graphically-derived source data elements, each graphically-derived source data element thereby being a graphically-derived version of a different source data element in the first data record, wherein identifying the desired source data element comprises selecting one of the plurality of graphically-derived source data elements as the desired source data element.

*This approach facilitates automated or semi-automated processing of the first data record in order to produce graphically-derived versions of source data elements. This approach avoids a need for direct access to the first data record in order to obtain or acquire data or information for copying to the desired destination data record.*

*Each of the plurality of graphically-derived source data elements therefore represents a potential source data element for selection as the desired source data element.* The step of processing the graphical representation of the first data record to produce a plurality of graphically-derived source data elements may comprise processing the graphical representation to identify a first set of label-value pairs, each label-value pair comprising a label for a graphically-derived source data element and a value for the graphically-derived source data element.

*Label-value pairs provide a specific implementation example for establishing data to be copied and*/*or for identifying the relevant source data element for a desired destination data element. In particular, a label part of a label value pair can be used to identify the relevant source data element to be copied to a particular destination data element (or vice versa), and the value part of the label value pair can contain the data to be copied to the destination data element.*

*This approach thereby provides a mechanism for facilitating automated identification of associated data elements and for improved accuracy in transferring relevant data therebetween.*

In some examples, the method further comprises a step of processing a graphical representation of the second data record to produce a plurality of graphically-derived destination data elements, each graphically-derived destination data element thereby being a graphically-derived version of a different destination data element in the second data record, wherein identifying the desired destination data element comprises selecting one of the plurality of graphically-derived destination data elements as the desired destination data element.

*Each of the plurality of graphically-derived destination data elements therefore represents a potential destination data element for selection as the desired destination data element.*

In some embodiments, the step of processing the graphical representation of the second data record to produce a plurality of graphically-derived destination data elements comprises processing the graphical representation to identify a second set of second label-value pairs, each second label-value pair comprising a label for a graphically-derived destination data element and a value for the graphically-derived destination data element. The step of selecting one of the plurality of graphically-derived destination data elements as the desired destination data element may comprise selecting the graphically-derived destination data element associated with a label that semantically corresponds to the label associated with the selected graphically-derived source data element.

The step of identifying the desired destination data element may comprise receiving a second user input indicating the desired destination data element. *This provides flexibility for a user to select and*/*or identify a desired destination data element.*

In some examples, the second user input is an input responsive to a single interaction between a user and a graphical representation of the second data record. *This reduces a complexity or difficulty for a user to select the desired destination element, e.g., by performing a single interaction with a graphical representation of the second data record. Thus, an improved human-machine interaction is achieved.*

In some examples, the single interaction is a click or press of a graphical representation of the desired destination data element in the graphical representation of the second data record.

The step of processing information in the first data record and information in the second data record may comprise comparing subject identifying information in the first data record to subject identifying information in the second data record to predict whether or not the first and second data records relate to a same clinical subject. *This approach uses subject information to perform the check as to whether the data records relate or pertain to a same subject. Subject information is information that is likely to be common to multiple data records, but is unlikely to change between different data records for a same individual. This facilitates determination, with a reasonable degree of certainty (in clinical terms), that the data records pertain to the same individual. For instance, is may be determined that the first and second data records relate to a same clinical subject only if the subject identifying information matches or differs only immaterially (e.g., the presence or absence of diacritics, the inclusion of middle names vs. no middle names and so on).*

The subject identifying information may include one or more of: a subject name, a subject identification number and/or a subject birth date. *These provide suitable examples of subject identifying information that is likely to be present across multiple data records for the subject, but is unlikely to change between different data records.*

There is also provided a computer program product comprising computer program code means which, when executed on a computing device having a processing system, cause the processing system to perform all of the steps of any herein disclosed method.

There is also provided a processing system for transferring data from a source clinical information system to a destination clinical information system, the processing system being configured to: identify a desired source data element in a first data record in the source clinical information system; identify a desired destination data element in a second data record in the destination clinical information system, wherein the desired source data element contains data for transferal to the desired destination data element; process information in the first data record and information in the second data record to predict whether or not the first and second data records relate to a same clinical subject; responsive to predicting that the first and second data records relate to the same clinical subject, copy data in the desired source data element to the desired destination data element; and responsive to predicting that the first and second data records do not relate to the same clinical subject, prevent the copying of data in the desired source data element to the desired destination data element.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 conceptually illustrates source and destination clinical information systems;
Fig. 2 is a flowchart illustrating a proposed method;
Fig. 3 is a flowchart illustrating another proposed method;
Fig. 4 is a flowchart illustrating yet another proposed method; and
Fig. 5 illustrates a system having a proposed processing system.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for transferring data from a first data record, in a source clinical information system, to a second data record, in a (different) destination clinical information system. A source data element, from the first data record, and a destination data element, from the second data record, are identified. A check is performed that both the first and second data records relate to a same clinical subject. Only if the check is passed is data transferred from the source data element to the destination data element.

Embodiments are based on the realization that there is a need for aided copying of information from a source data element to a destination data element, to avoid the time spent manually entering information, found in a first data record of a subject, in a second data record of a subject. This can reduce a burden on clinicians or other handlers of clinical information. The proposed approach resolves this need by providing an automated or semi-automated mechanism for copying data that also ensures that copied data pertains to a same clinical subject, to reduce the risk of inaccurate information being entered in the destination data element. This increase the safety of the clinical subject.

Proposed approaches can be performed in any clinical environment or clinical data analysis environment, but find particular use in hospitals or other busy clinical settings to reduce the burden on clinicians for completing data records for one or more clinical information systems.

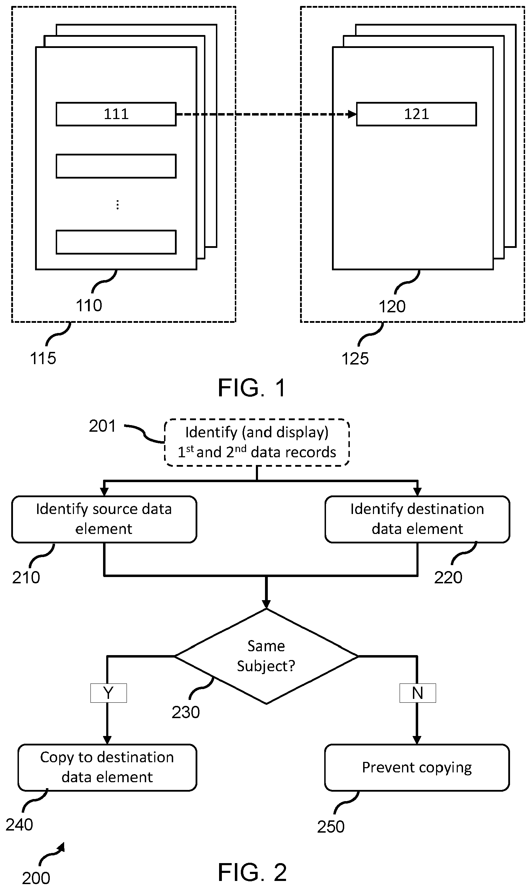

Fig. 1 conceptually illustrates a context in which embodiments of the present invention can be employed.

In particular, Fig. 1 conceptually illustrates a first data record 110 in a source clinical information system 115, e.g., which comprises a plurality of data records. Fig. 1 also conceptually illustrates a second data record 120 in a destination clinical information system 125, e.g., which also comprises a plurality of data records. The two information systems are different to one another, e.g., contain different sets of data records.

In practice, the first and second data records will be identified by a user, such as a clinician. In particular, the user may operate or initiate separate applications on a processing system with a user interface (such as a computer, tablet, laptop, mobile phone etc.) which is able to access or interact with different clinical information systems (e.g., for different vendors). In each application, the user may select, open or even create respective records for display, editing and/or completing. The selection, opening or creation of a data record may act as the identifying act of the relevant data record. In particular, the first and second data records are preferably data records that are displayed to the user.

The present invention relates to techniques for transferring data from the first data record 110 to the second data record 120. Thus, the present invention relates to techniques for transferring data between different clinical information systems 115, 125.

Put another way, proposed solutions facilitate the instantiation of a smart semantic communication (SSC) layer between foreground applications providing graphical representations of data records. This layer facilitates the data exchange between the foreground applications.

More specifically, proposed approaches provide a technique for transferring data contained or found in a desired source data element 111 to a desired destination data element 121. The desired source data element 111 is found in the first data record 110 (and therefore the source clinical information system 115). The desired destination source data element 121 is found in the second data record 120 (and therefore the destination clinical information system 125).

Fig. 2 illustrates a computer-implemented method 200 according to an embodiment.

The method 200 may comprise a step 201 of identifying the first and second data records, or a portion thereof.

The identity of the first and second data records may be selected or indicated by a user interacting with the user interface, e.g., selecting, viewing or otherwise interacting with the first and second data records. Approaches for selecting and displaying different data records at a same user interface are well known to the skilled person, e.g., by operating or initiating (via an input to the user interface) separate applications on a processing system that controls an output of the user interface.

Step 201 may comprise displaying the identified first and second data records, providing a graphical representation of the data records at a user interface, such as a screen, display or projector.

Thus, step 201 may comprise receiving an indication (via an initial user input) of the first and/or second data records and providing a graphical representation of the indicated first and second data records at a user interface.

The method 210 also comprises a step 210 of identifying a desired source data element.

A simple approach to performing step 210 is to receive a first user input identifying the desired source data element, e.g., by a user interacting with a (visual) representation of the desired source data element provided at a display. In this way, the first user input may, for instance, define which of a plurality of source data elements is selected as the desired source data element.

Preferably, the first user input is an input responsive to a single interaction between a user and a graphical representation of the first data record. For instance, the single interaction may be a click or press of a graphical representation of the desired source data element in the graphical representation of the first data record.

The computer-implemented method 200 also comprises a step 220 of identifying a desired destination data element.

A simple approach to performing step 210 is to receive a second user input identifying the desired destination data element, e.g., by a user interacting with a representation of the desired destination data element provided at a display. In this way, the second user input may, for instance, define which of a plurality of destination data elements is selected as the desired destination data element.

Preferably, the second user input is an input responsive to a single interaction between a user and a graphical representation of the second data record. For instance, the single interaction may be a click or press of a graphical representation of the desired destination data element in the graphical representation of the second data record.

This approach increases an ease for an individual or user in identifying the desired destination data element.

The method 200 also comprises a step 230 of processing information in the first data record and information in the second data record to predict whether or not the first and second data records relate to a same clinical subject.

Step 230 can be performed, for instance, by comparing subject identifying information in the first data record to subject identifying information in the second data record to predict whether or not the first and second data records relate to a same clinical subject. In particular, if there is a conflict or difference between the subject identifying information, then it may be determined that the two data records do not relate to the same clinical subject. Otherwise, the two data records may be considered to relate to the same clinical subject.

In some examples, minor differences in the subject-identifying information may be acceptable. For instance, minor variations in the subject name may be permitted. Such minor variations may include the presence or absence of diacritics in names or the like (which may reflect the storage mechanism of different clinical information systems).

The subject identifying information may include one or more of: a subject name, a subject identification number and/or a subject birth date. These provide suitable examples of data that is likely to be shared or consistent between different data records for a same subject or individual.

In one working example, step 230 comprises extracting subject identifying information from the first data record (or a graphical representation of the same) and the second data record (or a graphical representation of the same). Approaches for processing a graphical representation of a data record to discern data are later described, and may make use of optical character recognition.

Step 230 may then comprise comparing the extracted subject identifying information from the first and second data records. Step 230 may comprise determining whether or not the extracted information matches, e.g., is identical, to determine whether data records belong to a same subject.

The method 200 also comprises a step 240 of, responsive to predicting that the first and second data records relate to the same clinical subject, copying data in the desired source data element to the desired destination data element. In this way, the data in the desired source data element is copied over to the desired destination data element responsive to the data elements belonging to data records of a same subject.

The method 200 also comprises a step 250 of, responsive to predicting that the first and second data records do not relate to the same clinical subject, preventing the copying of data in the desired source data element to the desired destination data element. In this way, the data in the desired source data element is prevented from being copied over to the desired destination data element responsive to the data elements belonging to data records of different subject.

Step 250 may, for instance, comprise triggering a user-perceptible alert that the data records do not relate to a same subject. This can cause or prompt the user or clinician to understand that the identified data records may not correlate to one another, and correct the issue (e.g., by correcting a selection of the first and/or second data record - and thereafter reselecting the desired source/destination data element). The user-perceptible alert may, for instance, be provided at a same user interface to which a graphical representation of the first and/or second data records is provided.

A conflict in step 230 can be resolved or overridden by a user input. Thus, step 230 may comprise, responsive to determining that the two data records do not relate to the same clinical subject, prompting a user or individual to perform a check on the two data records. This may allow a user to override, e.g., via an override input, a decision by the method 200 to prevent copying of data.

The present invention provides techniques for avoiding, or at least reducing a likelihood of, data being inaccurately transferred between data records when they do not relate to a same subject. This reduces a risk of error in performing clinical analysis or diagnosis, as it significantly reduces a risk of inaccurate data being copied across.

Put another way, the proposed solution enables (semi-)automatic import and quality checks of data from any source clinical information system into a destination clinical information system. It starts with identifying (e.g., by a user indication) a data element X in the destination system that needs to be filled (such as the most recent procedure performed, or the blood pressure measurement), and the corresponding data element Y in the source system.

To guarantee that the right patient has been selected in both, a check of subject-identifying data elements will be performed. The subject-identifying data elements may, for instance, include at least: patient name and other identifiers, titles and contents of specific clinical data elements.

The system will therefore check whether the source and destination systems show data about the same subject and, if the check is passed, copy the contents of the source data element into the destination data element. This will ensure data consistency and quality and requires less effort from the individual.

The method 200 can be repeated, e.g., multiple instances performed simultaneously or sequentially, for different source and destination data elements.

It is appreciated that, in some circumstances, access to the first data record may be restricted. For instance, a user may only have "read-only" access to the first data record, e.g., to prevent unintentional editing. In circumstances such as these, it may not be possible to directly copy data found in the first data record to the second data record.

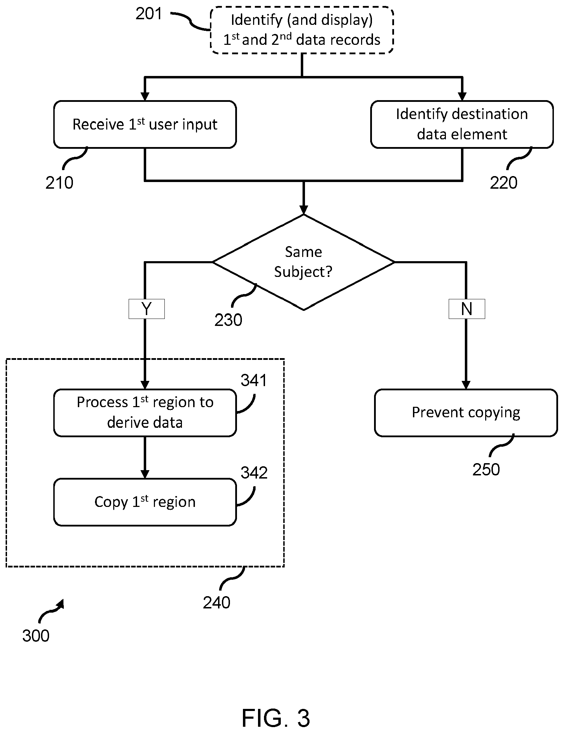

Fig. 3 illustrates a computer-implemented method 300 that seeks to overcome at least some of these concerns. However, the skilled person would appreciate how the proposed concept could be applied even if access to the first data record is available.

It will be apparent that the method 300 is a particular embodiment of the more generic method 200 described in Fig. 2. Thus, for the sake of conciseness, only those elements that differ from the previously described method will be hereafter described.

The method 300 is configured such that the step 210 of identifying the desired source data element comprises receiving a first user input. The first user input identifies or indicates the desired source data element.

More particularly, the first user input identifies or indicates a region of a graphical representation of the first data record (or a portion thereof) that includes a graphical representation of the desired source data element.

In the context of the present disclosure, a graphical representation is a data format or structure that is suitable for being graphically displayed. Thus, a graphical representation may comprise or contain display data that defines data provided to a display (such as a screen, monitor or projection).

Thus, identifying the desired source data element may comprise identifying a visual representation of the desired source data element. It will be appreciated that an identification of a visual representation of the desired source data element is functionally equivalent to an identification of the desired source data element itself, as both contain information suitable for identifying the desired source data element.

The method 300 is configured such that the step 240 of copying data in the desired source data element comprises processing 341 the indicated first region to produce a first graphically-derived version of the data in the desired source data element; and copying 342 the first graphically-derived version of the data into the desired destination data element.

In this way, the data that is copied to the desired destination data element is derived from a graphical representation of the desired source data element.

The method 300 effectively provides a technique in which an intermediary application or layer is able to relay information between two different applications that represent information from different clinical information systems. In particular, the intermediary application does not need to directly access the first data record in order to provide information to the second data record - rather, the information can be copied from a graphical representation of the first data record.

Step 341 may be performed by processing the indicated region using an optical character recognition (OCR) technique. This facilitates extraction of data contained in the first data record without needing to directly access the first data record, i.e., as indirect access is achieved through OCR processing of a graphical representation of the first data record.

Of course, if the graphical representation of the first data record is in the form of a drawing or other graphical object such as a graph or chart (e.g., rather than a text-based display), then a copy of the drawing or other graphical object may be made for copying to the desired destination data element in step 342.

Step 341 may be accordingly adapted to determine whether or not the data to be copied comprises text or not, and selectively applying an OCR technique only when it is determined that that the data to be copied comprises text. Thus, step 341 may comprise a step of determining whether or not the graphical representation of the desired source data element is text or a graphical object (such as a drawing, chart or graph). OCR may only be performed if the graphical representation contains text.

Other approaches for processing a graphical representation of information to extract information suitable for copying to a data record will be readily apparent to the skilled person, and may be employed in various embodiments of the proposed approach.

In the context of the present disclosure, data in a data element may comprise and/or be stored in the form of a label-value pair, also known as a key-value pair. A label-value pair contains a label for information (the "label"), e.g., in the form of textual data, and the information itself (the "value"), which may take the form of textual data or another form of data such as image data.

Thus, at the data record level, each data element contains a label-value pair, as each data element will contain a label for information (the label) and the information itself (the value).

The following passage(s) describes an approach for processing data in order to produce one or more label-value pairs - i.e., a "label-value pair generation process". In particular, the described approach provides a technique for processing graphically-derived data (e.g., data produced using an OCR procedure) to generate one or more label-value pairs.

Generating one or more label-value pairs is useful for automated identification or extraction of data to be copied to the desired destination data element for any herein described embodiment. In particular, inclusion of a label in the copied data may be extraneous (as the desired destination data element may already contain or be associated with a label). Thus, the label-value pair generation process may be used to identify the relevant data to be copied across (i.e., the value part of the label-value pair).

The same label-value pair generation process is also useful for automated identification or segmentation of data elements, e.g., from graphically-derived versions of a data record and/or a portion of a data record. This can be useful for later described embodiments. As one example, a portion of the first data record for copying to the second data record may be selected or identified by an individual, and it may be necessary to identify the separate data elements to be copied over. As another example, a desired destination data record may be identified by an individual, and a graphical representation of a first data record may need to be processed to identify the corresponding source data element containing the relevant data to be copied over. As yet another example, a graphical representation of a first data record may be processed to identify label-value pairs that represent source data elements that contain data to be copied over to a plurality of different destination data elements in the second data record. Examples of these and other techniques will be later described.

The label-value pair generation process may be used to form part of a smart semantic communication layer between two applications, i.e., between two clinical information systems. In particular, the label-value pair generation process may be able to regularize information in different clinical information systems.

The label-value pair generation process may comprise analyzing textual data extracted from a graphical representation of a data record (e.g., using OCR) to produce label-value pairs.

One approach for processing textual data to produce label-value pairs includes using a dictionary or vocabulary to identify values associated with labels or labels (e.g., "Name" or "Medications"). Data following such labels or labels (e.g., before reaching another label) is likely to be value data associated with the label.

Another approach for processing textual data is to monitor for change within the textual data. Change is only likely to occur for value data (whereas labels remain constant), such that value data can be readily identified.

The label-value pair generation process may comprise performing a spatial and/or graphical analysis on a graphical representation of a data record (e.g., using OCR) to produce label-value pairs. For instance, data found in certain locations (e.g., above boxes) of the graphical representation may represent labels, where data found in other locations (e.g., within boxes) may represent information associated with said labels. As another example, data found in bold text may represent labels, whereas data found in non-bold text may represent information associated with a label.

Moreover, examples of extracting label-value pairs (or key-value pairs) from unstructured data, such as that produced by OCR, is known in the art.

By way of example, one technique is proposed by Chung, Jeanhee, and Shawn Murphy. "Concept-value pair extraction from semi-structured clinical narrative: a case study using echocardiogram reports." AMIA Annual Symposium Proceedings. Vol. 2005. American Medical Informatics Association, 2005.

Another technique is proposed by Xie, Jiadong, et al. "Research on Structured Information Extraction Method of Electronic Medical Records of Traditional Chinese Medicine." 2020 IEEE International Conference on Bioinformatics and Biomedicine (BIBM). IEEE, 2020.

The above examples explain how different techniques could be used to produce label-value pairs from raw data and/or graphically-derived data.

This analysis results in the production of a set of label-value pairs, where a label is the label of the information and a value is the information itself. One set of label-value pairs will be created for each data record, and may be adapted when the information changes.

For each label-value pair, the semantics of each label and the semantics of each value can be extracted, e.g., using from label vocabularies, ontologies for clinical radiology (ICD, SNOMED, RadLex, ..) and so on. By way of example, for a lung nodule the label "Disorder of lung" found in SNOMED will result in a list of terms expected in the value. It is therefore possible to reverse this process and identify semantics associated with information found in the "value" of a label-value pair.

In some examples, each label-value pair is verified. This can be performed, for instance, by determining a probability value for each label-value pair. The probability value indicates the predicted relationship between the label and the value, and can be calculate by processing the semantics of the label and semantics of the value. A probability value of 0.0 may indicate no relation and a probability value of 1.0 may mean means it is predicted that the information in the label summarizes the contents of the value. For label-value pairs having a probability number less than a predetermined value (e.g., 0.8, 0.85, 0.9, 0.95 or 1.0), separate verification can then be performed by a user (e.g., by a user interacting with a user interface).

Label-value pairs of different data records can be correlated or associated with one another using the extracted semantics. In particular, a semantics of a label (in one data record) can be used to identify the label having corresponding or similar semantics in another data record.

After label-value pairs have been associated with one another, it is possible to automatically exchange data between the label-value pairs. In this way, the value of one label-value pair (of a first data record) can be copied to the value of another label-value pair (of a second data record).

It is possible to create or identify semantic equivalents to the contents of a label in a label-value pair of a first data set, e.g., using one or more dictionaries and/or thesauruses. In some embodiments, semantic equivalency may be determined between words of different languages (e.g., using suitable language dictionaries). A second set of label-value pairs may then be searched to identify a label-value pair (of the second set) having a label whose contents match the label or its semantic equivalents of the label-value pair of the first data set.

Label-value pairs are also useful for extracting subject-identifying information. In particular, a label in a label value pair may indicate whether the corresponding value relates to relevant subject-identifying information. If label-value pairs are produced for a data record, then the step 230 of determining whether the data records correspond to a same subject may comprise searching the label-value pairs to identify the subject-information. This may be performed using a keyword search of the labels, and extracting the value associated with any identified label(s). One of more semantic and/or language dictionaries may be used to identify labels having an equivalent meaning, i.e., associated with a particular desired piece of subject-identifying information.

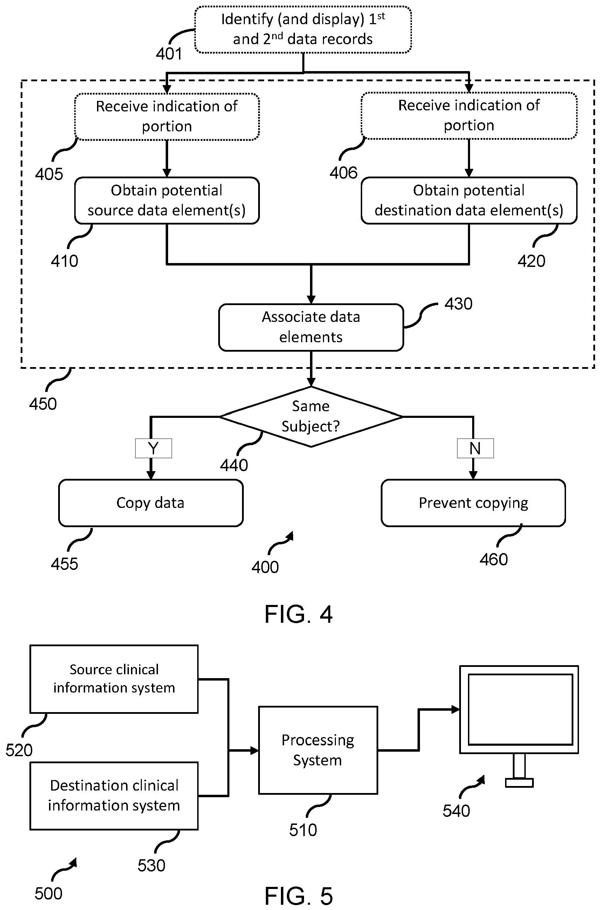

Fig. 4 illustrates a computer-implemented method 400 according to an embodiment.

The method 400 may comprise a step 401 of identifying the first and second data records, or a portion thereof.

The identity of the first and second data records may be selected or indicated by a user interacting with the user interface, e.g., selecting the first and second data records. Approaches for selecting and displaying different data records at a same user interface are well known to the skilled person, e.g., by operating or initiating (via an input to the user interface) separate applications on a processing system that controls an output of the user interface.

Step 401 may comprise displaying the identified first and second data records, providing a graphical representation of the data records at a user interface, such as a screen, display or projector.

Thus, step 401 may comprise receiving an indication (via an initial user input) of the first and/or second data records and providing a graphical representation of the indicated first and second data records at a user interface.

The method 400 comprises a procedure 450 for identifying at least one desired source data element and at least one desired destination data element. Each desired source data element corresponds or is associated with a different desired destination data element.

The procedure 450 comprises a step 410 of identifying one or more potential source data elements. Each potential source data element may be represented or take the form of a label-value pair.

In one example, each potential source data element is directly extracted from or identified within the first data record, e.g., contains all source data elements contained in the first data record.

In another example, each potential source data element is identified by a user input, e.g., identifying a selection of source data elements contained in the first data record. Thus, the set of potential source data elements may not contain all of the source data elements contained in the first data record.

In yet another example, each potential source data element is identified by processing a graphical representation of the first data record (or a portion thereof) to produce one or more graphically-derived source data elements. Each graphically-derived source data element is thereby a graphically-derived version of a different source data element in the first data record.

As previously mentioned, the first set of label-value pairs may be extracted by processing a graphical representation of a portion (e.g., not all) of the first data record. The portion may be identified by a user-interaction with a graphical representation of the first data record, e.g., by the user highlighting or dragging a box over a portion the graphical representation. The portion of the graphical representation identified by the user may be the portion of the first data record.

Accordingly, method 400 may comprise a step 405 of receiving an indication of the portion of the graphical representation of the first data record or an indication of the portion of the first data record. The indication may identify, for instance, only a single one of the potential source data elements or a graphical representation of only a single potential source data element. In other embodiments, the indication indicates a plurality of potential source data elements or a graphical representation of a plurality of potential source data elements.

Where each potential destination data element is represented as a key-value pair, step 410 thereby produces a first set of one or more key-value pairs.

The procedure 450 also comprises a step 420 of identifying one or more potential destination data elements. Each potential destination data element may be represented or take the form of a label-value pair.

In one example, each potential destination data element is directly extracted from the second data record, e.g., contains all destination data elements contained in the second data record.

In another example, each potential destination data element is identified by a user input, e.g., identifying a selection of destination data elements contained in the second data record. Thus, the set of potential destination data elements may not contain all of the destination data elements contained in the second data record.

In yet another example, each potential destination data element is identified by processing a graphical representation of the second data record (or a portion thereof) to produce one or more graphically-derived destination data elements. Each graphically-derived destination data element is thereby a graphically-derived version of a different destination data element in the second data record.

As previously mentioned, the first set of label-value pairs may be extracted by processing a graphical representation of a portion (e.g., not all) of the second data record. The portion may be identified by a user-interaction with a graphical representation of the second data record, e.g., by the user highlighting or dragging a box over a portion the graphical representation. The portion of the graphical representation identified by the user may be the portion of the second data record.

Accordingly, method 400 may comprise a step 406 of receiving an indication of the portion of the graphical representation of the second data record or an indication of the portion of the second data record. The indication may identify, for instance, only a single one of the potential destination data elements or a graphical representation of only a single potential destination data element. In other embodiments, the indication indicates a plurality of potential destination data elements or a graphical representation of a plurality of potential destination data elements.

Where each potential destination data element is represented as a key-value pair, step 420 thereby produces a second set of one or more key-value pairs.

The method 400 comprises a step 430 of associating each of one or more potential source data elements to a respective potential destination data element.

Step 430 may, for instance, be performed by using semantic correlation to identify similar data elements, e.g., by identifying semantic similarities between data contained in the data elements. This may make use of one or more semantic dictionaries and/or thesauruses. Such semantic dictionaries/thesaurus may identify semantic correlations between words of different languages.

In scenarios in which each data element is represented by a label-value pair, then step 430 can be performed by identifying semantic similarities between the contents of the labels of each label-value pair. This is advantageous when the value contains non-textual data, such as an image or figure. Approaches for performing this step have been previously described, and may make use of one or more semantic dictionaries and/or thesauruses.

In this way, step 430 effectively matches source data elements to destination data elements, i.e., associates source data elements with destination data elements.

Any source data element that becomes associated with a destination data element is effectively selected as a desired source data element. Similarly, any destination data element that becomes associated with a source data element is effectively selected as a desired destination data element.

The method 400 also comprises a step 440 of processing information in the first data record and information in the second data record to predict whether or not the first and second data records relate to a same clinical subject.

Step 440 may be performed by identifying any potential source and destination data elements that contain identifying information of a clinical subject (e.g., name, birthdate and so on). This can be performed using semantic identification techniques (e.g., using a semantic thesaurus or dictionary) that identify the relevant data elements based on their content. Where data elements are represented by label-value pairs, this can be performed by identifying the relevant label-value pairs based on the contents of the label in each label value pair.

The identifying information from the potential source data element(s) may then be compared to the identifying information from the potential destination data element(s) to predict whether or not the first and second data records relate to a same clinical subject, e.g., whether or not the identifying information matches.

The method 400 comprises, responsive to predicting that the first and second data records relate to the same clinical subject, a step 455 of copying data in the desired source data element(s) to the desired destination data element(s).

Where each data elements is represented by a key-value pair, step 455 may comprise, for each key-value pair in the first set of key-value pairs associated with a key-value pair in the second set of key-value pairs, copying the contents of the value portion of the key-value pair in the first set of key-value pairs to the value portion of the associated key-value pair in the second set of key-value pairs.

This provides a technique for automated copying of information from the first data record to the second data record.

The method 400 may comprises, responsive to predicting that the first and second data records relate to the same clinical subject, a step 460 of preventing the copying data in the desired source data element(s) to the desired destination data element(s).

The different embodiments illustrated by Fig. 2 to 4 effectively recognize that there are multiple potential levels of automation that make use of proposed concepts.

In particular, at a basic level, an individual may indicate or identify a single data element X (from the first data record) and a single data element Y (from the second data record), and the system may copy the contents from data element X to data element Y if the data records relate to the same subject. This is the embodiment illustrated by Figs. 2 and 3, and Fig. 4 (where an indication of only a single data record or graphical representation of a single data record is provided in both steps 405 and 406).

At another level an individual may indicates or identify one or more (e.g., more than one) data element from the first data record and one or more (e.g., more than one) data element from the second data record. The system may then identify associated data elements and copy the contents across associated data elements if the data records relate to the same subject. This is the embodiment illustrated by Fig. 4 (wherein steps 405 and 406 are performed).

At another level, an individual may identify one or more data elements (e.g., a single data element) in either the first data record or the second data record and not identify any data elements in the other data record. The system may then identify any data element in the other data record that is associated with the individual-identified data elements of the first/second data record. This embodiment is illustrated by Fig. 4 (where an indication provided in step 405 or 406, but not both, identifies only a single data element). Thus, only one of steps 405 and 406 is omitted to achieve this embodiment.

At yet another level, the system finds all data elements that can be imported from the first data record to the second data record. This embodiment is illustrated by Fig. 4, when steps 405 and 406 are omitted.

Fig. 5 illustrates a system 500 according to an embodiment.

The system 500 comprises a processing system 510 that carries out any herein described method.

The system 500 may further comprise the source clinical information system 520 (which stores at least the first data record) and the destination clinical information system 530 (which stores at least the second data record). The processing system 510 may be communicatively coupled to these information systems in order to facilitate the exchange of information from the source clinical information system to the destination clinical information system, i.e., by carrying out any herein described method.

The system 500 may comprise a user interface 540 configured to provide a visual representation of the first and/or second data record. In particular, the user interface may display a graphical representation of the first and/or second data record. Thus, the user interface may comprise a screen, monitor or projector for providing a visual representation of the first and/or second data records.

The user interface 540 may facilitate user interaction with the graphical representation(s) of the first and/or second data records in order to allow user identification of desired source/destination data element(s), e.g., by identifying one or more graphical representations of the same. Suitable elements for facilitating such user interaction are well known, and may make use of any user input device such as a mouse, keyboard, touchscreen, trackpad and so on.

The skilled person would be readily capable of developing a processing system 510 for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method.

There is also proposed a non-transitory storage medium that stores or carries a computer program or computer code that, when executed by a processing system, causes the processing system to carry out any herein described method.

In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa. Any reference signs in the claims should not be construed as limiting the scope.

## Claims

1. A computer-implemented method for transferring data from a source clinical information system to a destination clinical information system, the computer-implemented method comprising:
identifying a desired source data element in a first data record in the source clinical information system;
identifying a desired destination data element in a second data record in the destination clinical information system, wherein the desired source data element contains data for transferal to the desired destination data element;
processing information in the first data record and information in the second data record to predict whether or not the first and second data records relate to a same clinical subject;
responsive to predicting that the first and second data records relate to the same clinical subject, copying data in the desired source data element to the desired destination data element; and
responsive to predicting that the first and second data records do not relate to the same clinical subject, preventing the copying of data in the desired source data element to the desired destination data element.

2. The computer-implemented method of claim 1, wherein the step of identifying the desired source data element comprises receiving a first user input indicating the desired source data element.

3. The computer-implemented method of claim 2, wherein:
the first user input indicates a first region, of a graphical representation of the first data record, that graphically represents the desired source data element; and
the step of copying data in the desired source data element comprises:
processing the indicated first region to produce a first graphically-derived version of the data in the desired source data element; and
copying the first graphically-derived version of the data into the desired destination data element.

4. The computer-implemented method of claim 2, wherein the step of processing the indicated region comprises processing the indicated region using an optical character recognition technique.

5. The computer-implemented method of any of claims 1 or 2,
further comprising a step of processing a graphical representation of the first data record to produce a plurality of graphically-derived source data elements, each graphically-derived source data element thereby being a graphically-derived version of a different source data element in the first data record,
wherein identifying the desired source data element comprises selecting one of the plurality of graphically-derived source data elements as the desired source data element.

6. The computer-implemented method of claim 5, wherein the step of processing the graphical representation of the first data record to produce a plurality of graphically-derived source data elements comprises:
processing the graphical representation to identify a first set of label-value pairs, each label-value pair comprising a label for a graphically-derived source data element and a value for the graphically-derived source data element.

7. The computer-implemented method of claim 5 or 6,
further comprising a step of processing a graphical representation of the second data record to produce a plurality of graphically-derived destination data elements, each graphically-derived destination data element thereby being a graphically-derived version of a different destination data element in the second data record,
wherein identifying the desired destination data element comprises selecting one of the plurality of graphically-derived destination data elements as the desired destination data element.

8. The computer-implemented method of claim 7, only when dependent upon claim 6, wherein:
the step of processing the graphical representation of the second data record to produce a plurality of graphically-derived destination data elements comprises processing the graphical representation to identify a second set of second label-value pairs, each second label-value pair comprising a label for a graphically-derived destination data element and a value for the graphically-derived destination data element; and
the step of selecting one of the plurality of graphically-derived destination data elements as the desired destination data element comprises selecting the graphically-derived destination data element associated with a label that semantically corresponds to the label associated with the selected graphically-derived source data element.

9. The computer-implemented method of any of claims 1 to 8, wherein the step of identifying the desired destination data element comprises receiving a second user input indicating the desired destination data element.

10. The computer-implemented method of claim 9, wherein the second user input is an input responsive to a single interaction between a user and a graphical representation of the second data record.

11. The computer-implemented method of claim 10, wherein the single interaction is a click or press of a graphical representation of the desired destination data element in the graphical representation of the second data record.

12. The computer-implemented method of any of claims 1 to 11, wherein the step of processing information in the first data record and information in the second data record comprises comparing subject identifying information in the first data record to subject identifying information in the second data record to predict whether or not the first and second data records relate to a same clinical subject.

13. The computer-implemented method of claim 12, wherein the subject identifying information includes one or more of: a subject name, a subject identification number and/or a subject birth date.

14. A computer program product comprising computer program code means which, when executed on a computing device having a processing system, cause the processing system to perform all of the steps of the method according to any of claims 1 to 13.

15. A processing system for transferring data from a source clinical information system to a destination clinical information system, the processing system being configured to:
identify a desired source data element in a first data record in the source clinical information system;
identify a desired destination data element in a second data record in the destination clinical information system, wherein the desired source data element contains data for transferal to the desired destination data element;
process information in the first data record and information in the second data record to predict whether or not the first and second data records relate to a same clinical subject;
responsive to predicting that the first and second data records relate to the same clinical subject, copy data in the desired source data element to the desired destination data element; and
responsive to predicting that the first and second data records do not relate to the same clinical subject, prevent the copying of data in the desired source data element to the desired destination data element.
